Europäisches Patentamt

**European Patent Office**

**Office européen des brevets**

(19)

(11) Publication number: **0 161 018**

**A1**

(12) # EUROPEAN PATENT APPLICATION

(21) Application number: **85200522.2**

(22) Date of filing: **03.04.85**

(51) Int. Cl.⁴: **A 61 B 3/14**
**A 61 B 5/02, A 61 B 3/16**

(30) Priority: **11.04.84 NL 8401159**

(43) Date of publication of application:
**13.11.85 Bulletin 85/46**

(84) Designated Contracting States:
**DE FR GB NL**

(71) Applicant: **N.V. Optische Industrie "De Oude Delft"**
**Van Miereveltlaan 9**
**NL-2612 XE Delft(NL)**

(72) Inventor: **Schulte, Augustinus Victor M.C.**
**Parijseplein 25**
**NL-3332 SL Zwijndrecht(NL)**

(72) Inventor: **Vlasbloem, Hugo**
**Burgemeester van der Lelykade 4**
**NL-3155 AB Maasland(NL)**

(72) Inventor: **Beckmann, Leo Heinrich J.F.**
**Willem de Merodestraat 9**
**NL-2624 LC Delft(NL)**

(74) Representative: **van der Beek, George Frans, Ir. et al,**
**Nederlandsch Octrooibureau P.O. Box 29720**
**NL-2502 LS The Hague(NL)**

(54) **Device for retinal choroidal angiography or haematotachography.**

(57) Device for retinal or choroidal angiography or haematotachography, provided with a light source (1) which transmits light of specific wavelength(s) via a first optical path in the direction of the eye (6) and means which receive the light coming from the retina or choroid via a second optical path for observation (11) or recording (12) thereof. A dye which reacts to said light of specific wave length(s) is introduced into the bloodstream during use of said device. The device is provided with means (15, 16) by which, shortly before the dye-containing blood reaches the eye, the eye pressure is increased to above the (systolic) pressure in the arteria ophthalmica, and said pressure increase is removed after the dye concentration in the blood of the arteria ophthalmica has reached a predetermined value. Thereafter the means for observing or recording the light received are being put into operation.

fig-1

1

Device for retinal or choroidal angiography or haematotachography

The invention relates to a device for retinal or choroidal angiography or haematotachography, provided with a light source which transmits light of specific wavelength(s) via a first optical path in the direction of the eye and means which receive the light coming from the retina or choroid via a second optical path for observation or recording thereof, a dye which reacts to said light of specific wavelengths(s) being introduced into the bloodstream during use of said device.

A device of this type is, for example, known in the form of a so-called fundus camera, which is supplied by, inter alia, the West German firm of Zeiss. The observation or recording means in it are in the form of a photographic camera or television camera. The invention to be described below is indeed not limited to fundus cameras, but also extends to similar devices.

Such devices are used, inter alia, for studying the blood flow in the retinal and choroidal blood vessels. For this purpose, a fluorescent material (fluorescein) is generally introduced into the bloodstream. If light of specific wavelength(s) is beamed into the eye, the fluorescein in the blood vessels of the retinal and choroidal tissues will be activated and this fluorescein will start to emit light whose wavelength(s) differ(s) from that of the excitation light. In order to obtain an image of only those structures in the eye which contain fluorescein, two filters or groups of filters are generally used. The first filter, the excitation filter, is built into the optical path from the light source to the eye, and this filter allows through approximately only that part of the spectrum transmitted by the light source, by which the fluorescing of the fluorescein is activated. The second filter, the barrier filter, is built into the optical path between the eye and the camera and allows only fluorescent light to pass through. The wavelength of the fluorescent light is longer than that of the excitation light and, thanks to the two filters, only the fluorescent light can reach the photographic material in the camera part of the device.

Although with the known device, for example designed in the form of a fundus camera, a series of successive photographs can be taken, such photographs do not provide any information on the flow rate of the blood through the retinal and choroidal blood vessels. It is only the presence or absence of fluorescein which can be established at a given moment. The intensity of the fluorescent light will, however, depend also on the concentration of fluorescein in the blood. Since fluorescein is introduced into the bloodstream far away from the eye and does not reach the arteria ophthalmica – the artery behind the eye where the central retina artery (arteria centralis retinae) branches off – until after passing through the heart and lungs, there will be considerable dilution, and the fluorescein concentration in the capillary vessels of the eye will increase only slowly.

The object of the invention then is to indicate in what way and by what means a sharp front can be created between dye-containing blood and non-dye-containing blood, said front being maintained right into the capillary vessels.

This object is achieved with a device of the type mentioned in the preamble, in that the device is provided with means by which, shortly before the dye-containing blood reaches the eye, the eye pressure is increased to above the (systolic) pressure in the arteria ophthalmica, and said pressure increase is removed after the dye concentration in the blood of the arteria ophthalmica has reached a predetermined value, the means for observing or recording the light received also being put into operation.

The increasing of the eye pressure means that at the same moment the arteria centralis retinae is pressed shut at the point where it enters the eye, i.e. at the point where the greatest pressure gradient prevails. However, the blood in the arteria ophthalmica continues to flow through, and the dye concentration in the blood will increase quickly in it until the desired concentration is reached. At that moment the pressure increase is removed, so that the dye-containing blood flows into the arteria centralis retinae with a sharp front. This sharp front is maintained right into the capillary vessels. Since, simultaneously with the removal of the pressure increase, the camera is put into operation to take a series of photographs of the retina, a dynamic picture of the flow in the retinal vessels is obtained.

In an embodiment of the device according to the invention, the above-mentioned eye-pressure-increasing means are formed by an ophthalmodynamometer provided with means for indentation of the sclera, or provided with a suction cup which is placed on the sclera. Such means are known per se. Within the framework of the invention, these means are then used in a controlled manner to increase the eye pressure temporarily and therefore to prevent the admission of dye-containing blood for a certain period of time.

In one embodiment, prior to or simultaneously with the dye, a trigger substance is introduced into the bloodstream and the presence of the trigger substance in a peripheral part of the blood vessel system is detected with means suitable for the purpose. As the trigger substance, use can be made, for instance, of another fluorescent substance which as such has no adverse effect on the selective observation or recording of light coming from the actual dye.

The device according to the invention is preferably provided with a control unit which supplies the control signals to the eye-pressure-increasing means and which receives the detection signals from the dye detection means or the trigger substance detection means. As soon as the first dye reaches the relevant peripheral part of the blood vessel system, a signal is generated with this control unit to activate the eye-pressure-increasing means, so that at that moment the arteria centralis retinae is pressed shut at the point where it enters the eye and the flow of blood through the eye is temporarily blocked. As a result, at the point where the arteria centralis retinae branches off from the arteria ophthalmica a sharp front is produced between dye-containing blood and non-dye-containing blood, and this front can move into the capillaries inside the eye via the arteria centralis retinae as soon as the temporary eye pressure increase is removed.

As soon as the fluorescein has reached that part of the blood vessel system inside the eye which is irradiated by the excitation light, the fluorescein itself will start to emit light and this light can then be recorded using, for example, a camera. For this purpose, use can be made of a cine camera, with which photographic material is exposed, or use can be made of a video camera with a video recorder to obtain a recording on magnetic tape.

Instead of, or possibly in conjunction with, the dye detection means in the peripheral part of the blood vessel system, it is also possible to have the means receiving the light from the retina or choroid giving out a signal on the first detection of the fluorescent light, said signal then being used to increase the eye pressure via the means intended for the purpose. It would, for example, be conceivable to use a light-sensitive detector in the camera which gives off a signal on detecting the slightest fluorescent light. It would also be conceivable to use with a video camera a detector to which the output signal of the video camera is fed and which gives off a signal when a very low threshold level is exceeded. This means that traces of fluorescein-containing blood can indeed already reach the eye, but its light yield is much too low to obtain a clear recording. In this case also a sharp wave front will form, preceded by blood with a very low fluorescein concentration. Here again, a sharp and also clear separation will remain between the high-dye blood and the low-dye blood, and a clear recording of the front will be possible.

Assuming a predetermined average blood flow velocity, the control unit can be designed in such a way that it is provided with timing means which are activated simultaneously with the emission of the activating signal to the eye-pressure-increasing means, said timing means after a predetermined period of time deactivating the eye-pressure-increasing means and also putting into operation the means for observing or recording the light received.

The invention will be discussed in greater detail below with reference to the attached figures.

Fig. 1 shows a schematic view of a device according to the invention provided with an optical system and eye-pressure-increasing means with a suitably shaped rod for indentation of the sclera.

Fig. 2 illustrates schematically the occurrence of the front between high-dye blood and low-dye blood or blood containing no dye at all.

Fig. 3a, 3b give details of the device during use.

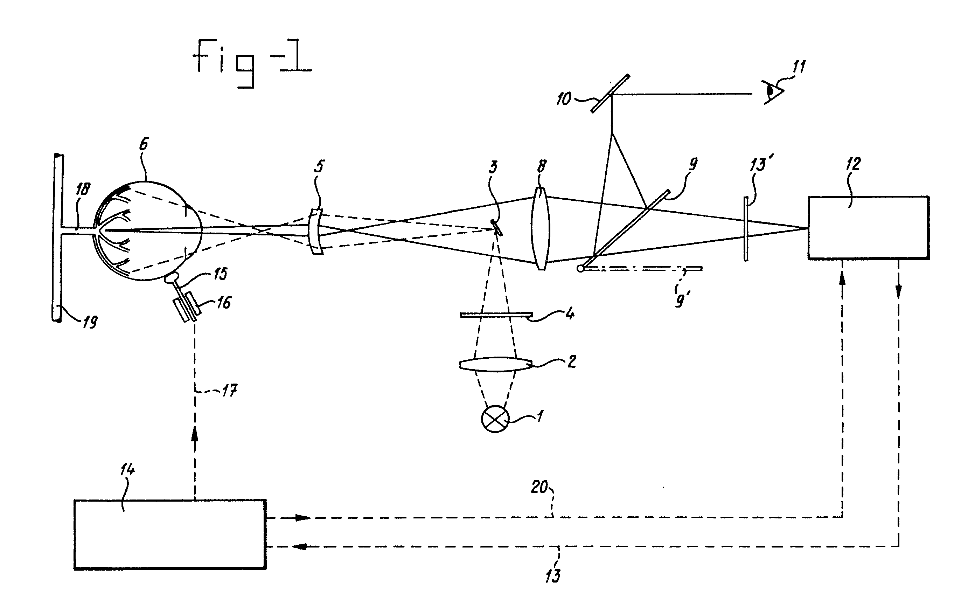

Fig. 1 illustrates schematically an embodiment of a device according to the invention. On the right-hand side is the actual camera part, combined with the excitation light source with further means. The light from the excitation light source 1 is directed via the lens 2 to the mirror 3. Located in this path also is the excitation filter 4. From the mirror 3 the beam of light is directed via the lens 5 to the eye 6, where it enters the retina or the choroid. If there is fluorescein in the choroidal or retinal blood vessels indicated schematically by 7, this fluorescein will be activated by the light beamed in and will itself start to emit fluorescent light. As shown by the solid lines in Fig. 1, this fluorescent light will be beamed in via the lens 5 and the lens 8 to the mirror 9, from where in the first instance it passes a further mirror 10 to reach the eye 11 of an observer. This optical light path can, for instance, be used to set the camera.

During the taking of a series of photographs, the mirror 9 will be folded downwards into the position indicated by 9', so that the light can reach the camera, only schematically shown by 12. In the path between the lens 8 and the camera 12 is the barrier filter 13, which allows only fluorescent light to pass through.

It is pointed out that instead of a movable mirror 9, it is also possible to use a semi-transparent mirror of the type that will be known to the expert.

In the case of a photographic camera 12, this camera 12 is, for example, provided with a detector which, so long as no fluorescent light is emitted, gives off a predetermined signal. As soon as the first fluorescein enters the eye, the output signal of this detector will change and from it will be derived a trigger signal which is fed via the connection 13 to a control unit 14. The control unit 14 at that moment delivers an activation signal to the indentation means, consisting of a schematically shown indentation rod 15, which is movable inside a coil 16. By means of the activation signal given off by the control unit 14 via the line 17 the coil 16 is excited in a manner not described in greater detail, so that the indentation rod 15 is moved over a predetermined short distance in the direction of the eye 6, in such a way that a pressure increase takes place in the eye 6, with the result that the arteria retina centralis 18 is pressed shut at the point where it enters the eye, the point where the greatest pressure gradient will exist, so that the flow through said arteria retina centralis 18 comes to a standstill. However, the blood in the arteria ophthalmica 19 lying behind it continues to flow through and the fluorescence concentration in it will quickly increase until a suitable concentration level is reached. In practice, it is possible, for example, to incorporate in the control unit 14 a timing unit, which measures a predetermined period of time which in practice is sufficient to obtain a high enough dye concentration directly behind the eye. At the end of this period the indentation rod 15 will be withdrawn so that the pressure increase in the eye 6 is suddenly removed, and the high-fluorescence blood can enter the retinal or choroidal vessels with a sharp wave front from the arteria ophthalmica 19 via the artery 18. As a result of the excitation light beamed into the eye, the fluorescein will be activated and will itself begin to emit light at a wavelength differing from that of the excitation light.

Simultaneously with the withdrawal of the indentation rod 15, the camera 12 is also put into operation with a signal which is given off via the line 20 by the control unit 14. The camera thus begins to take pictures of the dye-containing blood which flows with a sharp front right into the capillaries of the retina or choroid.

If as the camera 12 a video camera is used, it is, for example, also possible, instead of the above-mentioned detector, to feed the output signal from this camera to a threshold value switch which, so long as there is no fluorescein present in the eye yet, will give off an output signal at a constant level. However, as soon as the first fluorescein enters the eye, the video camera will record some light, with the result that the output signal increases. The threshold value switch responds to it immediately and then gives off the above-mentioned signal via the line 13 to the control unit 14, which reacts to it by moving the indentation rod 15.

As is known per se from the state of the art, the light source 1 can be of various types, for example a laser light source or flash light sources such as xenon lamps which can transmit light flashes at relatively high speed.

The parts 15 and 16, the indentation rod and the accompanying exciter coil, form part of an ophthalmodynamometer which is known per se. Such an ophthalmodynamometer is assumed to be known by the expert and will not therefore be discussed in detail. It is merely pointed out that instead of an indentation rod 15 use can also be made of a suction cup which is placed against the sclera of the eye, and which can also produce a pressure increase in the eye. If this suction cup is connected to a vacuum in relation to the existing atmospheric pressure, part of the sclera will be sucked into the suction cup and the eye thereby deformed, as a result of which the pressure in the eye rises. The use of such suction cups is, however, also known to the expert and requires no further discussion.

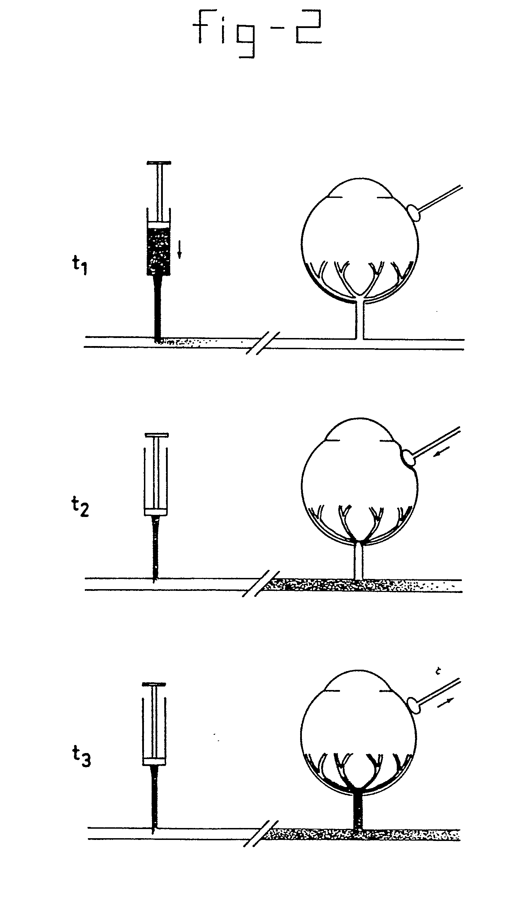

Fig. 2 illustrates in three phases the formation of a sharp front in the blood entering the eye.

At time $t_1$ fluorescein is injected into the bloodstream at a suitable place. The indentation rod of the ophthalmodynamometer is not yet activated.

At time $t_2$ the detection means have found that the dye has reached the peripheral part of the blood vessel system. On detection thereof, the indentation rod is activated so that the

0161018

pressure in the eye is increased and there is temporarily no flow of dye-containing blood into the arteria retina centralis.

As soon as the dye concentration in the arteria ophthalmica has increased sufficiently, the indentation rod is withdrawn at time $t_3$, as a result of which dye-containing blood enters the blood vessels in the eye with a clearly demarcated front in relation to non-dye-containing blood.

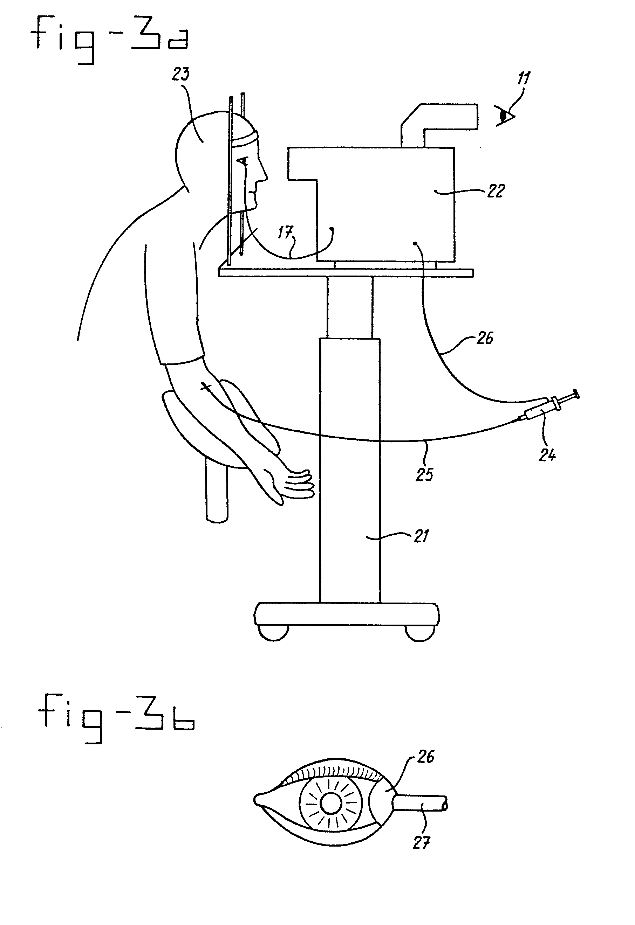

Figs. 3a and 3b give more detailed information concerning the practical use of the device according to the invention.

Resting on an underframe 21 is the device 22, which in principle comprises all parts 1 up to and including 10, 12 and 13. The control unit 14 can also be accommodated in it. As schematically shown, the head of the patient 23 is supported in a suitable manner at the front of the device 22 in such a position that the excitation light generated in the device 22 enters the patient's eye in the correct manner. Right beside the eye, fixed to the supporting structure of the entire device in a manner not illustrated in detail, is the indentation rod 15 with its exciter coil to which a control signal can be fed via the line 17. If a suction cup is used instead of the indentation device, the connection 17 is a flexible hose which is connected to the suction cup which is placed against the sclera. Via this hose, a vacuum can be produced in the suction cup for increasing the eye pressure.

By means of a syringe 24, fluorescein is injected either directly or via a tube 25 into the patient's bloodstream. In the example illustrated in Fig. 3a, the fluorescein is injected into the arm. At the same time as the syringe 24 is being operated, a signal is given off via the line 26 to the device 22, so that the detector present therein can be activated. As soon as the detector receives the first fluorescent light, the device 22 will become operative together with the ophthalmodynamometer in the way already described and, after a delay period in which the eye pressure is temporarily increased, a series of photographs can be taken, during which a sharp fluorescent wave front advances in the eye.

Fig. 3b indicates schematically the way in which a so-called suction cup can be used. The suction cup 26 is connected via a vacuum line 27 to vacuum means not shown in any greater detail. The suction cup is placed against the side of the eye outside the path of the light from the device 22 and is otherwise excited or not excited in the same way as the indentation rod by the control unit 14. The use of a suction cup is also known to the expert in this field.

Instead of a detector in the device 22, it is also possible to establish the arrival of the fluorescein in another part of the peripheral blood vessel system and to draw conclusions from it concerning the time of arrival of the fluorescein directly behind the eye. The arrival of fluorescein or of an individual trigger substance in the outermost part of the ear can, for example, be measured easily and accurately.

## CLAIMS

1. Device for retinal or choroidal angiography or haematotachography, provided with a light source which transmits light of specific wavelength(s) via a first optical path in the direction of the eye and means which receive the light coming from the retina or choroid via a second optical path for observation or recording thereof, a dye which reacts to said light of specific wave length(s) being introduced into the bloodstream during use of said device characterised in that the device is provided with means by which, shortly before the dye-containing blood reaches the eye, the eye pressure is increased to above the (systolic) pressure in the arteria ophthalmica, and said pressure increase is removed after the dye concentration in the blood of the arteria ophthalmica has reached a predetermined value, the means for observing or recording the light received also being put into operation.

2. Device according to Claim 1, characterised in that the above-mentioned eye-pressure-increasing means are formed by an ophthalmodynamometer provided with means for indentation of the eye or provided with a suction cup against the eye.

3. Device according to Claim 1 or, 2, characterised in that the device is provided with means for the detection of the presence of dye in a peripheral part of the blood vessel system.

4. Device according to any of the preceding claims, characterised in that prior to or simultaneously with the dye, a trigger substance is introduced into the bloodstream and the presence of the trigger substance in a peripheral part of the blood vessel system is detected with means suitable for the purpose.

5. Device according to any of the preceding claims, characterised in that the device is provided with a control unit which feeds control signals to the eye-pressure-increasing means, and which receives the detection signals from the dye detection means or the trigger substance detection means.

6. Device according to any of the preceding claims, characterised in that the light received from the retina or choroid is recorded with the aid of a cine camera on photographic material or with the aid of a video camera and a video recorder on magnetic tape.

7. Device according to any of the preceding claims, characterised in that the means which receive the light from the retina or choroid are provided with a detector which gives off a signal on the first detection of light, said signal being used to increase the eye pressure via the means intended for the purpose.

8. Device according to any of Claims 5 up to and including 7, characterised in that the control unit is provided with timing means which are activated simultaneously with the signal for the eye-pressure-increasing means, said timing means after a predetermined period of time deactivating the eye-pressure-increasing means and also putting into operation the means for observing or recording the light received.

*****

Fig -1

# fig-2

$t_1$

$t_2$

$t_3$

fig-3a

fig-3b

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| A | DE-A-3 117 699 (METRICON LTD.) * Abstract; page 11, lines 17-22; page 15, line 10 - page 16, line 23; page 17, lines 3-16; page 19, lines 4-13; figures 1,2,8,9 * | 1,3,7 | A 61 B 3/14<br>A 61 B 5/02<br>A 61 B 3/16 |
| A | DE-B-1 078 735 (F. SCHWARZER GmbH) * Column 1, line 20 - column 2, line 47; figures 1,2 * | 2,5,8 | |
| A | PHYSICS IN MEDICINE AND BIOLOGY, vol. 24. no. 3, May 1979, pages 489-504, London, GB; R.A. WEALE: "Physics and ophthalmology" * Pages 493- 496, paragraph 3: "Fluorescein angiography" * | 1,3,6 | |
| A | US-A-3 893 447 (B.F. HOCHHEIMER) * Abstract; column 2, lines 8-44; column 4, lines 48-67; column 6, lines 21-61; figures 1,2 * | 1,3,4,6 | TECHNICAL FIELDS SEARCHED (Int. Cl.4)<br><br>A 61 B<br>A 61 F |
| A | US-A-3 835 836 (Y.C. KANTER et al.) * Abstract; column 2, line 60 - column 3, line 18; column 4, line 31 - column 5, line 31; column 6, lines 47-56; figures 1,2 * | 1,2 | |

---    -/-

The present search report has been drawn up for all claims

| Place of search | Date of completion of the search | Examiner |
|---|---|---|
| THE HAGUE | 24-07-1985 | RIEB K.D. |

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,A | NL-A-8 301 049   (A.V.M.C.L. SCHULTE) | | |

----

TECHNICAL FIELDS
SEARCHED (Int. Cl.4)

The present search report has been drawn up for all claims

| Place of search | Date of completion of the search | Examiner |
|---|---|---|
| THE HAGUE | 24-07-1985 | RIEB K.D. |